# EUROPEAN PATENT APPLICATION

(11) **EP 4 467 177 A1**
(43) Date of publication of application: **27.11.2024**
(21) Application number: 24151408.2
(22) Date of filing: 11.01.2024
(51) Int. Cl.: A61M 5/24, A61M 5/28, A61M 5/31

(54) **CARTRIDGE AND DISPENSING DEVICE**

(71) Applicant: medmix Switzerland AG, 9469 Haag (Rheintal) (CH)
(72) Inventor: BASTOS, Emanuel, 9470 Buchs (CH)
(74) Representative: Manitz Finsterwald Patent- und Rechtsanwaltspartnerschaft mbB

(57) **Abstract**

The present invention relates to a cartridge for a dispensing device, comprising a housing defining an outer shape of the cartridge and a grip plate. Further, the present invention relates to a dispensing device comprising a cartridge, in particular the cartridge according to one of the preceding claims, the cartridge comprising a housing defining the outer shape of the cartridge and a grip plate.

## Description

### FIELD

The present invention relates to a cartridge for a dispensing device, comprising a housing defining an outer shape of the cartridge and a grip plate. Further, the present invention relates to a dispensing device comprising a cartridge, in particular said cartridge according to the present invention, the cartridge comprising a housing defining the outer shape of the cartridge and a grip plate.

### BACKGROUND

Cartridges and also dispensing devices comprising said cartridges exist in different forms and sizes. Syringes are especially simple dispensing devices, wherein in particular a syringe cylinder can be considered as a cartridge.

Further, cartridges can comprise grip plates for improving a handling of the overall dispensing device. Especially with syringes, but not limited to, one-piece solutions are provided, in which the grip plate is an integral part of the housing. As there is a need for variation of grip plates, for instance with respect to size, shape, position and/or orientation, this leads to many individual items, which in turn leads to increased costs in manufacture and storing. Also turning the dispensing device during usage, for instance in dental applications when changing from work at the upper jaw to work at the lower jaw, is hindered.

Accordingly, there is a need for cartridges and dispensing devices which provide an easy way to alter features of a grip plate of the cartridge.

### SUMMARY

The present disclosure provides a cartridge and a dispensing device according to the independent claims. Embodiments are given in the subclaims, the description and the drawings.

In one aspect, the present disclosure is directed at a cartridge for a dispensing device, comprising a housing defining an outer shape of the cartridge and a grip plate, wherein the grip plate is radially moveably mounted at the housing and axially fixable to the housing.

The housing of the cartridge according to the present invention in most of the cases extends from a first end of the cartridge up to a second end of the cartridge. By that, the outer shape of the cartridge is defined by the housing. Thereby, an axial direction and likewise a longitudinal axis of the cartridge and of the housing are identical.

Said first end of the cartridge can be for instance a dispensing end and as such preferably comprises connection means for an arrangement of a dispensing tip. Said connection means can in particular be provided by the housing.

Said second end of the cartridge can for instance comprise an opening for an insertion of a piston for dispensing material provided in the cartridge through the respective other end of the cartridge, namely the first end. Also, arrangement means for an arrangement of an external dispensing apparatus are possible. Said arrangement means can in particular be provided by the housing.

Additionally to the housing, the cartridge according to the present invention comprises a grip plate. The housing and the grip plate are accordingly constructed with respect to each other. In particular, the grip plate is mountable on the housing such that it is simultaneously on the one hand axially fixed and on the other hand radially movable.

Preferably, the grip plate is arrangeable reversibly on the housing.

Constructing the housing and the grip plate as two separate and distinct parts provides the advantage that, concerning a cartridge with a specific housing, the grip plate is exchangeable. By that, for different applications, the grip plate suited best can be selected. In addition, also for different users, different grip plates can be attached to the housing, for instance according to a hand size of the respective user.

As the grip plate is axially fixed to the housing, it can be used as a holding structure, for instance to manually exert an axial force on a piston used to dispense material stored in the cartridge.

Further, as the grip plate is simultaneously radially movably arranged at the housing, a rotation of the housing, and hence of the cartridge, is possible without lifting a grip on the grip plate. Especially with rotational unsymmetrically dispensing tips, such as for instance dispensing tips comprising a bended shape, this allows altering the application area without lifting the grip on the cartridge or even the need of changing the used cartridge.

According to an embodiment, an open internal cross section of the cartridge perpendicular to an axial direction of the cartridge is round, oval, or D-shaped. In the interior of the cartridge, one or more materials to be dispensed can be provided. For that purpose, the cartridge comprises at least one internal cavity for arranging said material, whereby said one or more cavity comprises the open internal cross section along to the axial direction of the cartridge. By providing said cross section in a round, oval, or D-shaped form, the requirements of a vast variety of materials to be dispensed can be met. In other words, by selecting the respective most suitable open internal cross section, the cartridge can be constructed for receiving different materials.

According to an embodiment, the cartridge comprises two containers arranged in parallel, the two containers having equal or unequal volumes and/or equal or unequal outer cross-sectional areas. As mentioned above, the cartridge provides material to be dispensed. However, alternatively to storing the material directly in a cavity formed by material of structural parts of the cartridge, such as for instance the housing, the material can also be provided in containers separated from said structural parts. By using two containers, dispensing a mixture of different materials, each stored in one of the containers, can be provided, for instance by using a dispensing tip comprising a mixing element. Preferably, said containers are reversibly arranged within the cartridge. By that, re-using the structural parts of the cartridge with different containers, even different containers providing different materials, is possible. However, also embodiments of the cartridge according to the present invention with a single container comprising the material to be dispensed are feasible.

According to an embodiment, an outer shape of the housing is round at least in sections along the axial direction of the cartridge. Preferably, the housing comprises a round shape with a constant cross section along the longitudinal direction of the cartridge. This provides an overall pen-like appearance of the cartridge, which in turn enhances a handling on the cartridge. In addition, a round shape of the housing facilitates the provision of the radial movability of the grip plate arranged at the housing.

According to an embodiment, the grip plate is axially locked into place at one end of the housing. Preferably, said one end is opposite to an end of the housing at which dispensing the material takes place. An especially good grip during a manual dispensing process, especially by pressing a piston into the cartridge, can be provided.

According to an enhanced embodiment, the grip plate is snap fit into place at the one end of the housing. Mechanically, a snap fit is an especially easy way of arranging the grip plate at the one end of the housing. For a snap fit, a respectively constructed snap fit part of the one of the elements, preferably of the grip plate, is elastic and is deflected during the arrangement process by a respectively constructed deflecting part of the respective other element. At the end of the process, the snap fit part snaps back into its previous shape and form-fittingly engages behind the deflection part. If, as in most cases, the mounting of the grip plate includes an axial movement of the grip plate along the housing, this automatically provides the axial fit of the grip plate on the housing.

According to an alternative or additional enhanced embodiment, said one end of the housing comprises a stepped structure to which the grip plate is axially locked. In other words, the stepped structure of the housing forms the deflection part as described in the previous paragraph. Additionally to a snap fit in its most simple embodiment, a stepped structure further provides an axial stop surface for the grip plate in general. Axial forces acting on the snap fit towards the stop surface, and hence towards the stepped structure, can thereby be lowered. Especially forces acting during an actual dispensing process are mostly transferred directly into the stepped structure and hence into the housing without exerting stress on the snap fit. Preferably, the stepped structure is provided annular on the housing.

According to an enhanced embodiment, the stepped structure comprises one or more undercuts. Said undercuts allow in an especially easy way that the respective part of the grip plate, introduced above as snap fit part, snaps behind the stepped structure into the one or more undercuts for form-fittingly fixating the grip plate on the housing with respect to an axial movement. Preferably, the one or more undercuts are provided in an annular manner. If applicable, said undercut and said axial stop surface are arranged on opposite axial ends of the stepped structure.

According to an embodiment, the grip plate comprises a one or more ribs. Ribs help to increase torsional rigidity of the grip plate. Preferably, said rib is provided as an annular structure on the grip plate.

According to an embodiment, one of said one or more ribs engages one of said one or more undercuts for axially locking the grip plate into place at said one end of the housing, in particular wherein said rib is sloped on at least one side to provide an insertion aid. According to this specific embodiment, one of said ribs is also involved in the above-described snap fit of the grip plate on the housing. The overall complexity of the grip plate can thereby be lowered, as said rip provides two functionalities, namely enhancing torsional rigidity and snap fit functionality. By providing the rib with a slope, the arrangement process of the grip plate on the housing can be simplified, as a deflection of the rib on the respective stepped structure requires less force.

According to an embodiment, the grip plate comprises an aperture for placement over the housing. In other words, the grip plate is circumferentially closed and encompasses the housing. During the arrangement process, the housing is inserted into the aperture, and the grip plate is moved along the housing till its designated position. Preferably, the aperture is accordingly shaped and sized with respect to the housing. Thereby, a linear movement of the grip plate perpendicular to the longitudinal direction of the housing can be limited, preferably avoided, without hindering the rotational movement of the grip plate.

According to an enhanced embodiment, the grip plate further comprises a collar surrounding said aperture. Said collar elongates the aperture along the longitudinal direction. Thereby, said collar enhances the advantages of the aperture described in the previous paragraph. In other words, the collar acts as a kind of bearing during the movement of the grip plate along the housing. Preferably, said collar comprises the same open cross section as the aperture.

According to an embodiment, the grip plate comprises at least two oppositely arranged handles, wherein preferably the at least two oppositely arranged handles are square-shaped or round-shaped. In particular, but not limited to, a usage of the cartridge in or as a syringe, in most of the cases the user holds the cartridge with index finger and middle finger, whereas the piston is actuated by the thumb of the user. Providing the grip plate with two or more oppositely arranged handles, said usage can be simplified. It has been found that square-shaped or round-shaped handles are most convenient for the user.

According to an embodiment, the grip plate is moveable between a first end of the housing and a second end of the housing along a longitudinal axis of the housing in predefined steps defined by a plurality of annular grooves in an outer surface of the housing, and the grip plate is fixable to the housing at any predefined step between the first end of the housing and the second end of the housing by receiving one or more protruding lips of the grip plate through one groove of the plurality of grooves. In particular, the second end of the housing can be identical to the one end of the housing mentioned above. In other words, the grip plate can be fixed at several axial positions on the housing along the longitudinal direction. In each of said axial positions, the one or more protruding lips engages into the respective groove and axially fixes the grip plate. By providing the grooves as annular grooves, the rotational movability of the grip plate can be provided in each of the aforementioned positions. The grooves of the plurality of grooves can be evenly spaced. However, also different distances between the grooves are possible. Providing the grip plate with an annular rib and/or an aperture and/or handles as described above is also possible. A cartridge with an axially fixed and nevertheless rotationally movable grip plate at different axial positions can thereby be provided.

In another aspect, the present disclosure is directed at a dispensing device comprising a cartridge, in particular the cartridge according to the present invention, the cartridge comprising a housing defining the outer shape of the cartridge and a grip plate, wherein the grip plate is radially moveably mounted at the housing and axially fixable to the housing.

The housing and the grip plate are provided as two separate and distinct parts. In other words, the grip plate is exchangeable. Hence, for different applications and/or users, the grip plate suited best can be selected. As the grip plate is axially fixed but nevertheless rotatably arranged at the housing, the grip plate can be used as a holding surface, e.g. to manually exert an axial force on a piston used to dispense material stored in the cartridge, and further a rotation of the cartridge is possible without lifting a grip on the grip plate. A dispensing device enhanced especially with respect to a cartridge with a one-piece housing comprising integral grip plates can thereby be provided.

Preferably, the dispensing device according to the present invention comprises a cartridge according to the present invention. All advantages described above with respect to different embodiments of the cartridge according to the present invention can also be provided by the dispensing device according to the present invention comprising the respective embodiment of the cartridge according to the present invention.

### DRAWINGS

Exemplary embodiments of the present disclosure are described herein in conjunction with the following drawings, showing schematically:
- Fig. 1: A side view of a part of a cartridge during an arrangement process of a grip plate,
- Fig. 2: A sectional view of the cartridge of Fig. 1 after completion of the arrangement process,
- Fig. 3: A 3D-view of a cartridge during an arrangement process of a grip plate,
- Fig. 4: A 3D-view of the cartridge of Fig. 3 after the arrangement process of the grip plate, and
- Fig. 5: A 3D-view of the cartridge of Fig. 4 with further rotated grip plate.

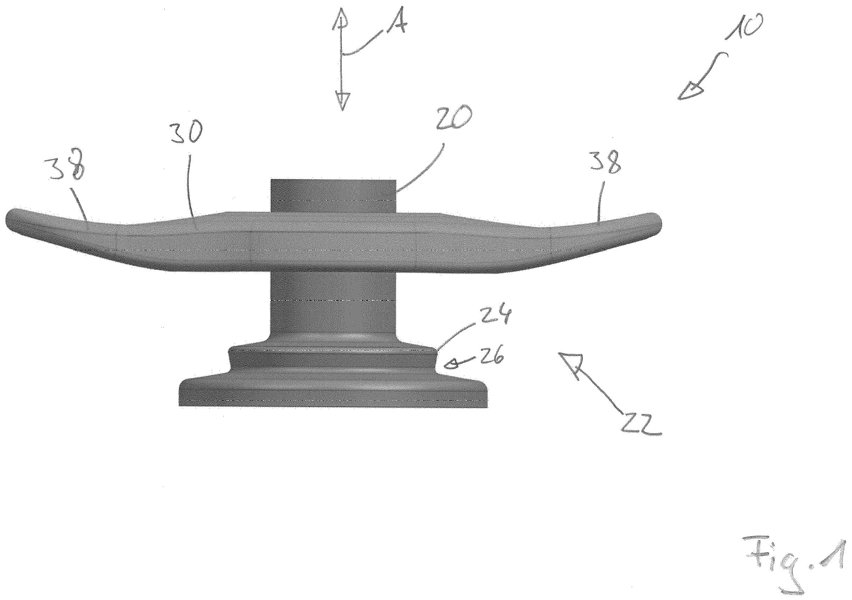

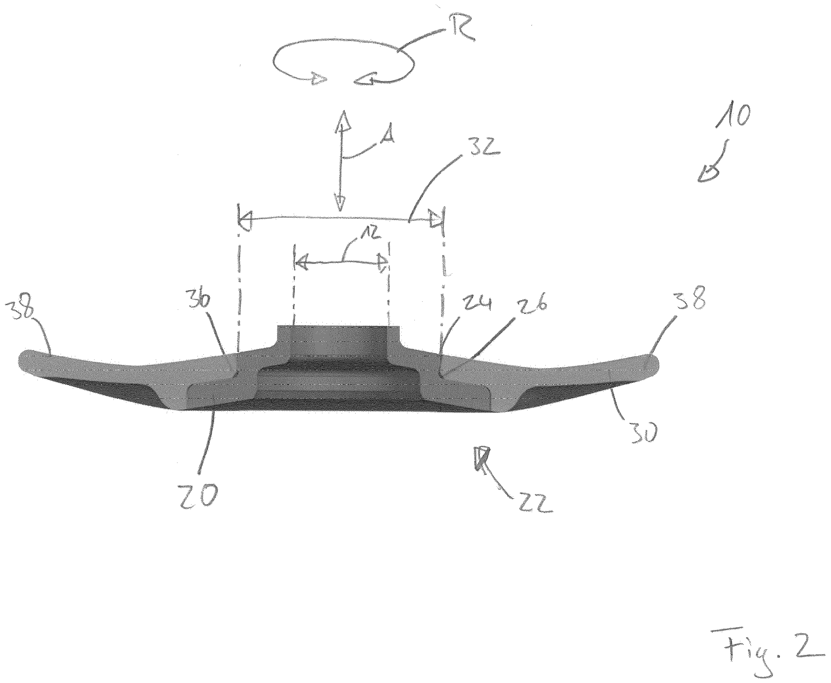

Fig. 1 and 2 depict a part of a cartridge 10 according to the present invention around an upper end 22 of the housing 20, and an arrangement process of a grip plate 30 at the housing 20. Fig. 1 shows the cartridge 10 during the arrangement process in a side view, Fig. 2 shows the cartridge 10 after completion of the arrangement process in a sectional view. In the following, Fig. 1 and 2 are described together.

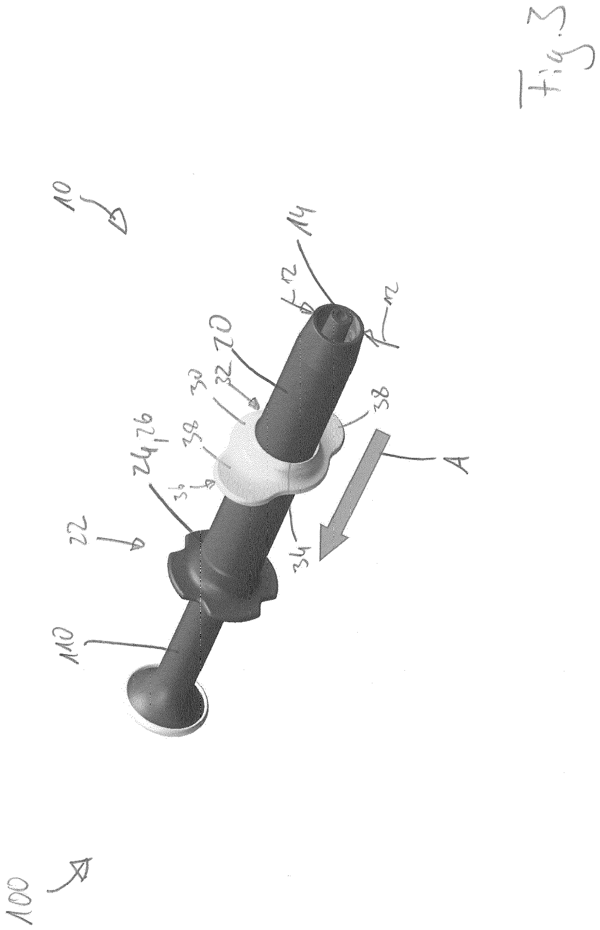

The housing 20 comprises cavity with an open internal cross section 12 (see Fig. 2), in which a material to be dispensed can be stored, either directly in the cavity or in one or more additional containers 14 (not depicted, see Fig. 3 to 5). The grip plate 30 enhances usability of the cartridge 10, in particular when the dispensing process is manually actuated. This is for instance the case when the cartridge 10 according to the present invention is used as part of a syringe.

The cartridge 10 according to the present invention comprises a housing 20 and a grip plate 30 as separate parts. During the arrangement process, the grip plate 30 slides over the housing 20 along the axial direction A of both the cartridge 10 and the housing 20 towards one end 22 of the housing 20 (see Fig. 1). For this, the grip plate 30 comprises an aperture 32 for allowing a placement of the grip plate 30 over the housing 20.

At said one end 22, the housing 20 comprises a stepped structure 24, which is preferably constructed with an undercut 26. When arriving at the one end 22 of the housing 20, a rib 36 formed at the grip plate 30 engages and hence snap fits into the undercut 26. Thereby, the grip plate 30 is axially fixed to the housing 20. For providing an insertion aid, the rib 36 can be sloped on one side, in particular on the side facing the one end 22.

As described in the previous paragraph, the grip plate 30 snap fits on the housing 20. However, this fixation is effective only in the axial direction A, in the rotational direction R (see Fig. 2) a movement of the grip plate 30 is still possible. In other words, after snap fitting the grip plate 30 is still able to rotate. In particular, the handles 38, which can be for instance square-shaped or round-shaped, can be positioned at any angular position with respect to the housing 20.

As depicted, the housing 20 preferably comprises a round outer shape. Especially this round outer shape allows to arrange the grip plate 30 in a radially movable way at the housing 20. However, the rotatability of the grip plate 30 is not limited to this general round outer shape of the housing 20.

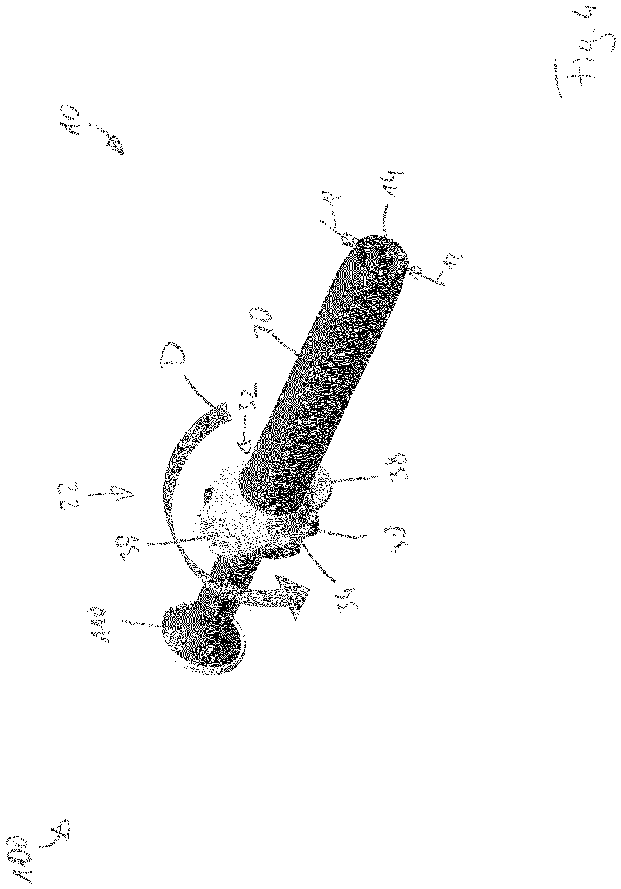

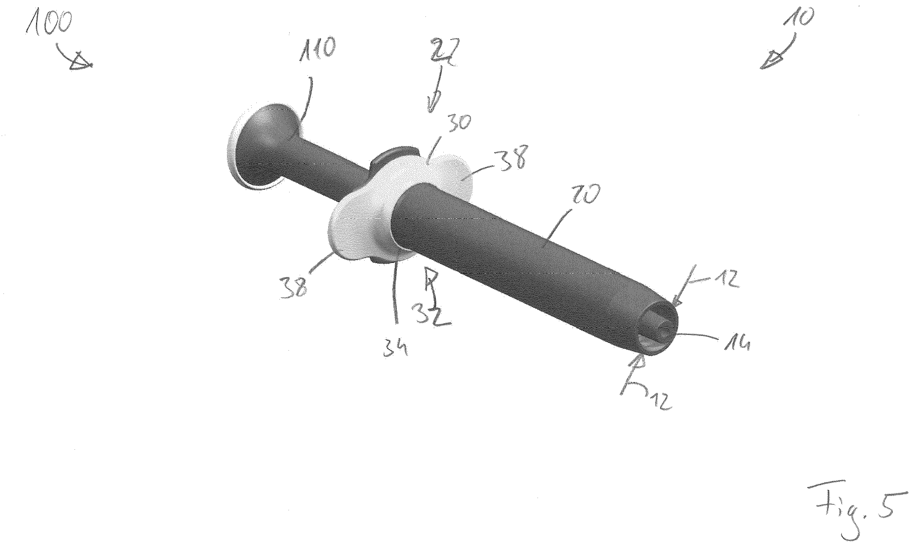

Fig. 3 to 5 depict a dispensing device 100 according to the present invention, which comprises a cartridge 10 according to the present invention. Fig. 3 shows the dispensing device 100 during the arrangement process of a grip plate 30 at a housing 20 of the cartridge 20, Fig. 4 and 5 show the dispensing device 100 after completion of said arrangement process, with the grip plate 30 in different rotational positions. In the following, Fig. 3 to 5 are described together.

Additional to the cartridge 10, the dispensing device 100 comprises a container 14 which is positioned in the internal open cross section 12 of the housing 20. In other embodiments, also two or more containers 14 are possible, which can have equal or unequal volumes and/or cross-sectional areas, and are arranged in parallel in a cavity forming the open cross section 12 of the cartridge 20. Depending on the embodiment, said internal open cross section 12 can be for instance round (see Fig. 3 to 5), oval or D-shaped. A piston 110 of the dispensing device 100 allows manually actuating the dispensing process.

The cartridge 10 of the depicted embodiment of the dispensing device 100 is constructed similar to the cartridge 10 depicted in Fig. 1, 2. Hence, reference is made to the description of these Fig. 1, 2 above.

Especially, also in the embodiment depicted in Fig. 3 to 5, the grip plate 30 and the housing 20 are provided as separate parts, wherein during assembling the grip plate 30 is positioned with its aperture 32 over the round outer shape of the housing 20 and slips along the axial direction A towards the one end 22 of the housing 20 (see Fig. 3). A collar 34 elongates the aperture 32 along the axial direction A and hence acts as a bearing both for the axial movement during the arrangement process of the grip plate 30, and also later on for the rotational movement of the grip plate 30 described in the following.

Upon arrival at the one end 22 (see Fig. 4), the grip plate 30 snap fits onto a stepped structure 24 of the housing 20. For that, the stepped structure 24 comprises an undercut 26 in which a rib 36 (indicated by an arrow in Fig. 3) of the grip plate 30 engages. This results in an axial fixation of the grip plate 30 at the one end 22 of the housing 20.

However, a rotational movement of the grip plate 30 is still possible. This is depicted in Fig. 4, 5. In Fig. 4, the grip plate 30 is depicted in a first rotational position during moving in a rotational direction R, in Fig. 5 at the end of said rotational movement in a second rotational position. As clearly visible, the handles 38 of the grip plate 30 are positioned in different angles with respect to the housing 20 of the cartridge 10. Likewise, a rotation of the housing 20 is possible while maintaining the handles 38, and hence the grip plate 30, in a spatially fixed position.

In the embodiments of the cartridge 10 depicted in Fig. 1 to 5, the grip plate 30 is axially fixed at the one end 22 of the housing 20. According to an alternative and not depicted embodiment, also a movement of the grip plate 30 along longitudinal direction A at the housing 20 and a fixation of the grip plate 30 at several predefined positions along said movement is possible. For that, a plurality of annular grooves is provided in an outer surface of the housing 20, and the grip plate 30 comprises one or more protruding lips for engaging into one of said annular grooves. Thereby, the grip plate 30 is fixable to the housing 20 at any predefined step between the first end of the housing 20 and the second end of the housing 20 by receiving the one or more protruding lips of the grip plate 30 through the respective one groove of the plurality of grooves.

### Reference numeral list

- 10: cartridge
- 12: internal cross section
- 14: container
- 20: housing
- 22: one end
- 24: stepped structure
- 26: undercut
- 30: grip plate
- 32: aperture
- 34: collar
- 36: rib
- 38: handle
- 100: dispensing device
- 110: piston
- A: axial direction
- R: rotational direction

## Claims

1. A cartridge (10) for a dispensing device (100), comprising
a housing (20) defining an outer shape of the cartridge (10) and a grip plate (30),
wherein the grip plate (30) is radially moveably mounted at the housing (20) and axially fixable to the housing (20).

2. The cartridge (10) of claim 1,
wherein an open internal cross section (12) of the cartridge (10) perpendicular to an axial direction (A) of the cartridge (10) is round, oval, or D-shaped.

3. The cartridge (10) of at least one of claims 1 or 2,
wherein the cartridge (10) comprises two containers (14) arranged in parallel, the two containers (14) having equal or unequal volumes and/or equal or unequal outer cross-sectional areas.

4. The cartridge (10) of at least one of claims 1 to 3,
wherein an outer shape of the housing (20) is round at least in sections along the axial direction (A) of the cartridge (10).

5. The cartridge (10) of at least one of claims 1 to 4,
wherein the grip plate (30) is axially locked into place at one end (22) of the housing (20).

6. The cartridge (10) of claim 5,
wherein the grip plate (30) is snap fit into place at the one end (22) of the housing (20).

7. The cartridge (10) of claim 5 or claim 6,
wherein said one end (22) of the housing (20) comprises a stepped structure (24) to which the grip plate (30) is axially locked.

8. The cartridge (10) of claim 7,
wherein the stepped structure (24) comprises one or more undercuts (26).

9. The cartridge (10) of at least one of claims 1 to 8,
wherein the grip plate (30) comprises one or more ribs (36).

10. The cartridge (10) of claim 8 and claim 9,
wherein one of said one or more ribs (36) engages one of said one or more undercuts (26) for axially locking the grip plate (30) into place at said one end (22) of the housing (20), in particular wherein the rib (36) is sloped on at least one side to provide an insertion aid.

11. The cartridge (10) of one of claims 1 to 10,
wherein the grip plate (30) comprises an aperture (32) for placement over the housing (20).

12. The cartridge (10) of claim 11,
wherein the grip plate (30) further comprises a collar (34) surrounding said aperture (32).

13. The cartridge (10) of at least one of claims 1 to 12,
wherein the grip plate (30) comprises at least two oppositely arranged handies (38), wherein preferably the at least two oppositely arranged handles (38) are square-shaped or round-shaped.

14. The cartridge (10) of at least one of claims 1 to 13,
wherein the grip plate (30) is moveable between a first end of the housing (20) and a second end of the housing (20) along a longitudinal axis of the housing (20) in predefined steps defined by a plurality of annular grooves in an outer surface of the housing (20), and the grip plate (30) is fixable to the housing (20) at any predefined step between the first end of the housing (20) and the second end of the housing (20) by receiving one or more protruding lips of the grip plate (30) through one groove of the plurality of grooves.

15. A dispensing device (100) comprising a cartridge (10), in particular the cartridge (10) according to one of the preceding claims, the cartridge (10) comprising a housing (20) defining the outer shape of the cartridge (10) and a grip plate (30), wherein the grip plate (30) is radially moveably mounted at the housing (20) and axially fixable to the housing (20).
